# EUROPEAN PATENT SPECIFICATION

(11) **EP 4 285 951 B1**
(45) Date of publication and mention of the grant of the patent: **30.04.2025**
(21) Application number: 22176393.1
(22) Date of filing: 31.05.2022
(51) Int. Cl.: A61M 1/00

(54) **NASAL ASPIRATOR CAPABLE OF EFFECTIVELY PREVENTING BACKFLOW AND CONTROL METHOD THEREOF**
NASALER ASPIRATOR, DER FÄHIG IST, EINEN RÜCKFLUSS ZU VERHINDERN, UND STEUERUNGSVERFAHREN DAFÜR
ASPIRATEUR NASAL CAPABLE D'EMPÊCHER EFFICACEMENT LE REFLUX ET SON PROCÉDÉ DE COMMANDE

(43) Date of publication of application: 06.12.2023
(73) Proprietor: Hetaida Technology Co., Ltd., Dongguan City, Guangdong 523000 (CN)
(72) Inventor: Chen, Zhenguang, Dongguan City, Guangdong 523000 (CN)
(74) Representative: Cabinet Chaillot

(56) References cited:
- CN-U- 211 272 649
- TW-B- I 754 566
- US-A1- 2017 095 599
- US-A1- 2022 296 800

## Description

### BACKGROUND OF THE INVENTION

### 1. Field of the Invention

The present invention relates to a nasal aspirator, and more particularly to a nasal aspirator capable of effectively preventing backflow and a control method thereof.

### 2. Description of the Prior Art

A nasal aspirator is a special tool to suck the nasal secretions of a baby because the baby is too small to blow his/her nose by himself/herself. When the nasal secretions are dry to form booger to block the nasal passageway, it is difficult for the baby to breathe.

There are four types of nasal aspirator on the market, namely, a pump-type nasal aspirator, a mouth-suction-type nasal aspirator, a spray-type nasal aspirator and a steam-type nasal aspirator. The pump-type nasal aspirator is easy to use and carry and can suck out nasal mucus and secretions. As to the mouth-suction-type nasal aspirator, the suction can be adjusted by the user, the length of the suction tube is suitable, the small and flexible suction head will not extends into the inside of the nasal cavity, and it is easy to use. The spray-type nasal aspirator and the steam-type nasal aspirator are generally professional medical nasal aspirators. The more high-end nasal aspirator for home use is what we often call an electric nasal aspirator. It is running by "suction", but the energy supply is "electricity".

A conventional electric nasal aspirator mainly relies on a built-in air pump unit to generate suction for extracting the secretions in the nasal cavity into a liquid storage cup of the electric nasal aspirator. In the prior art, due to various reasons such as the overfilling of the liquid storage cup, the liquid is likely to flow back into the air pump unit to cause damage to the air pump unit and to cause inconvenience to the user. An electric nasal aspirator having a liquid detection function to automatically stop an air pump is developed on the market. However, the airway of this electric nasal aspirator is designed too simply and unreasonably. It is triggered too early, resulting in shutdown of the air pump unit. It is inconvenient to use. Therefore, it is necessary to study a solution to solve the above problems.

TW I 754 566 B discloses a nasal aspirator according to the preamble of claim 1.

CN 211 272 649 U discloses a nasal aspirator wherein a liquid detection system is provided to detect liquid overflowing from a liquid storage cup.

### SUMMARY OF THE INVENTION

In view of the deficiencies of the prior art, the primary object of the present invention is to provide a nasal aspirator capable of effectively preventing backflow and a control method thereof, which can effectively solve the problem that the existing electric nasal aspirator is easy to cause backflow of nasal secretions into the air pump unit or that the air pump unit is easily triggered to cause shutdown.

In order to achieve the above object, the present invention adopts the following technical solutions.

A nasal aspirator, according to claim 1, comprises a casing, a control board, a lower suction nozzle, an upper suction nozzle, a liquid storage cup, and an air pump unit. The control board is disposed in the casing. The lower suction nozzle is disposed on an upper end of the casing. The lower suction nozzle has a chamber and a suction passageway therein. The chamber has an opening facing upward. An upper end of the suction passageway communicates with the chamber. A detection system for detecting a liquid is provided in the chamber. The detection system is connected to the control board. The upper suction nozzle is mounted to an upper end of the lower suction nozzle. The upper suction nozzle includes a main body shell, an inner tube, and an outer tube. A lower end of the main body shell is hermetically connected to the upper end of the lower suction nozzle. The main body shell has a cavity with an opening facing downward. The cavity communicates with the chamber. The inner tube extends from a top wall of the cavity. The inner tube has a first passageway. The outer tube extends upward from an upper end face of the main body shell. The outer tube has a second passageway. The second passageway communicates with the first passageway. The liquid storage cup is placed in the chamber and the cavity. The liquid storage cup includes a cup body and a cup cover. The cup body has an accommodating chamber with an opening facing upward. The cup cover is disposed on the cup body to cover the opening of the accommodating chamber. The cup cover is formed with a through hole and a mounting hole. The through hole communicates with the accommodating chamber and the cavity. The inner tube passes through the mounting hole and extends into the accommodating chamber. An outer wall of the inner tube is hermetically mated with an inner wall of the mounting hole. The air pump unit is disposed in the casing. An air intake end of the air pump unit communicates with a lower end of the suction passageway. The air pump unit has a motor. The motor is connected to the control board.

A control method of the foregoing nasal aspirator is provided. After the control board is powered on, the air pump unit is turned on and the motor starts to run. The air pump unit generates a suction to suck air in the suction passageway, the chamber, the cavity, the through hole, the accommodating chamber, the first passageway and the second passageway in sequence. When an upper end of the upper suction nozzle is placed in a user's nasal cavity, secretions in the nasal cavity flow through the third passageway, the second passageway and the first passageway in sequence under the action of the suction and flow into the accommodating chamber to be collected. When the motor runs, the control board controls the detection system to detect in real time whether there is a liquid flowing into the chamber of the lower suction nozzle for performing backflow detection. If the liquid is detected, the control board controls the motor to stop running, or, the motor continues to run for collecting the secretions.

Compared with the prior art, the present invention has obvious advantages and beneficial effects. Specifically, it can be known from the above technical solutions:
By providing the detection system in the cavity, the detection system can detect whether there is a liquid in real time, so as to control the motor of the air pump unit. When the liquid is detected, the motor stops running, which effectively prevents the liquid from backflow to enter the air pump unit. Therefore, the air pump unit is well protected, avoiding damage to the air pump unit and greatly prolonging the service life of the product. The airway of the nasal aspirator is composed of the suction passageway, the chamber, the cavity, the through hole, the accommodating chamber, the first passageway and the second passageway. The airway is designed more reasonably, which effectively avoids shutdown of the air pump unit because the detection system is triggered too early. It is convenient to the use.

### BRIEF DESCRIPTION OF THE DRAWINGS

FIG 1 is a perspective view according to a preferred embodiment of the present invention;
FIG 2 is an exploded view according to the preferred embodiment of the present invention;
FIG 3 is another exploded view according to the preferred embodiment of the present invention;
FIG 4 is a cross-sectional view according to the preferred embodiment of the present invention; and
FIG 5 is a partial cross-sectional view according to the preferred embodiment of the present invention when in use.

### DETAILED DESCRIPTION OF THE PREFERRED EMBODIMENTS

Embodiments of the present invention will now be described, by way of example only, with reference to the accompanying drawings.

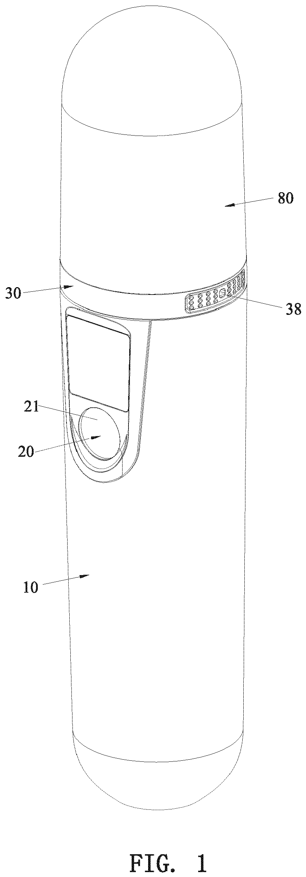

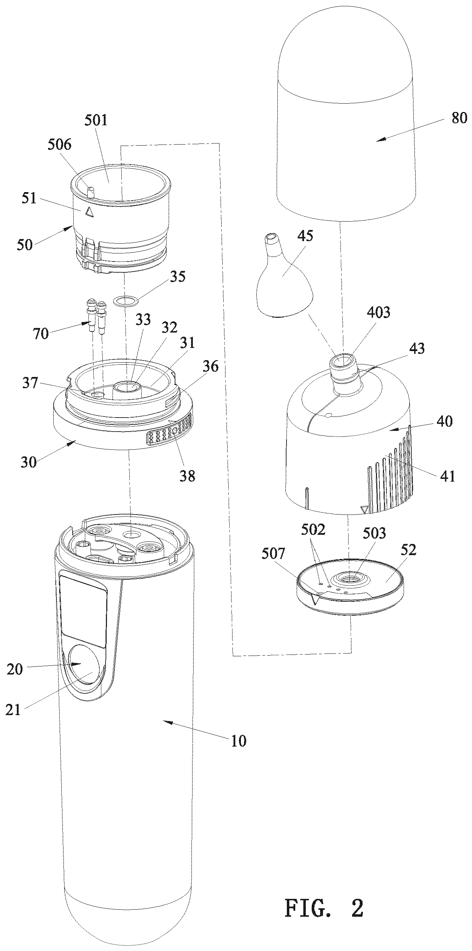

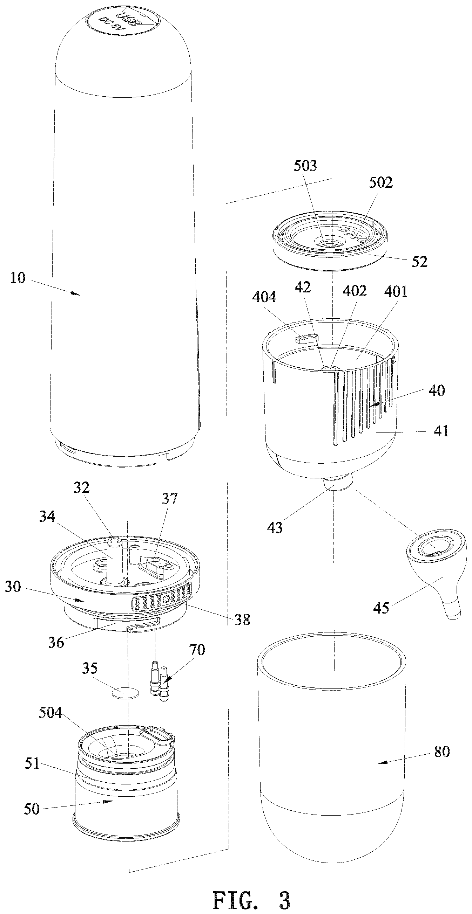

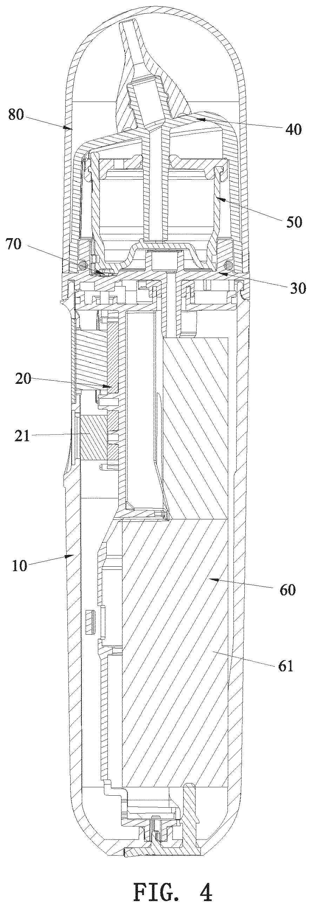

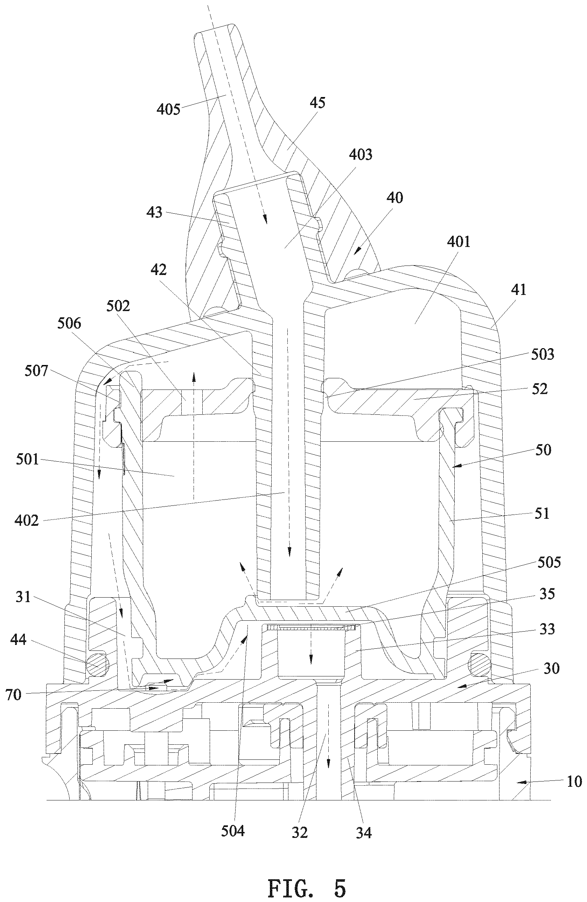

Referring to FIG 1 to FIG 5, a nasal aspirator capable of effectively preventing backflow according to a preferred embodiment of the present invention comprises a casing 10, a control board 20, a lower suction nozzle 30, an upper suction nozzle 40, a liquid storage cup 50, and an air pump unit 60.

The control board 20 is disposed in the casing 10. The control board 20 has a control button 21. The control button 21 is exposed to the casing 10. The control board 20 may be connected to an external power supply or directly connected to a built-in power supply to supply power to the control board 20.

The lower suction nozzle 30 is disposed on the upper end of the casing 10. The lower suction nozzle 30 has a chamber 31 and a suction passageway 32 therein. The chamber 31 has an opening facing upward. The upper end of the suction passageway 32 communicates with the chamber 31. A detection system 70 for detecting a liquid is provided in the chamber 31. The detection system 70 is connected to the control board 20. In this embodiment, the lower suction nozzle 30 has a first boss 33 extending from the bottom of the chamber 31. The lower suction nozzle 30 further has a connecting tube 34 extending downward from the bottom of the lower suction nozzle 30. The suction passageway 32 is formed in the first boss 33 and the connecting tube 34. A waterproof and breathable membrane 35 is fixed on the upper end face of the first boss 33. The waterproof and breathable membrane 35 covers the upper opening of the suction passageway 32, so as to prevent liquid from flowing into the suction passageway 32 effectively. The outer wall of the upper end of the lower suction nozzle 30 is recessed with an arc-shaped engaging groove 36. The bottom of the chamber 31 is formed with a fixing hole 37. The fixing hole 37 extends downward and passes through the bottom of the lower suction nozzle 30. The detection system 70 is a probe. The probe is inserted and secured in the fixing hole 37. The detection system 70 includes two detection systems 70 connected to the control board 20. The outer wall of the lower suction nozzle 30 is formed with a vent 38 communicating with the inside of the casing 10 for the air pump unit 60 to exhaust air.

The upper suction nozzle 40 is mounted to the upper end of the lower suction nozzle 30. The upper suction nozzle 40 includes a main body shell 41, an inner tube 42, and an outer tube 43. The lower end of the main body shell 41 is hermetically connected to the upper end of the lower suction nozzle 30. The main body shell 41 has a cavity 401 with an opening facing downward. The cavity 401 communicates with the chamber 31. The inner tube 42 extends from the top wall of the cavity 401. The inner tube 42 has a first passageway 402. The outer tube 43 extends upward from the upper end face of the main body shell 41. The outer tube 43 has a second passageway 403. The second passageway 403 communicates with the first passageway 402. In this embodiment, the lower end of the main body shell 41 is sleeved on the upper end of the lower suction nozzle 30. A sealing ring 44 is sandwiched between the inner wall of the lower end of the main body shell 41 and the outer wall of the upper end of the lower suction nozzle 30. An engaging portion 404 extends from the inner wall of the lower end of the main body shell 41. The engaging portion 404 is rotatably engaged with the arc-shaped engaging groove 36. The outer tube 43 is provided with a detachable suction head 45. The suction head 45 has a third passageway 405 therein. The third passageway 405 communicates with the second passageway 403. The upper suction nozzle 40 is provided with a detachable cover 80.

The liquid storage cup 50 is placed in the chamber 31 and the cavity 401. The liquid storage cup 50 includes a cup body 51 and a cup cover 52. The cup body 51 has an accommodating chamber 501 with an opening facing upward. The cup cover 52 is disposed on the cup body 51 to cover the opening of the accommodating chamber 501. The cup cover 52 is formed with a through hole 502 and a mounting hole 503. The through hole 502 communicates with the accommodating chamber 501 and the cavity 401. The through hole 502 includes a plurality of spaced through holes to facilitate air intake. The inner tube 42 passes through the mounting hole 503 and extends into the accommodating chamber 501. The outer wall of the inner tube 42 is hermetically mated with the inner wall of the mounting hole 503. In this embodiment, the outer bottom surface of the cup body 51 has a recess 504. A second boss 505 is formed on the inner bottom surface of the cup body 51. The first boss 33 is located in the recess 504. The inner tube 42 is close to the second boss 505. The lower opening of the first passageway 402 faces the upper surface of the second boss 505. The cup cover 52 is coupled to the cup body 51. A positioning post 506 extends upward from the upper end of the cup body 51. The cup cover 52 has a positioning hole 507. The positioning post 506 is inserted through the positioning hole 507 and extends out of the cup cover 52.

The air pump unit 60 is disposed in the casing 10. The air intake end of the air pump unit 60 communicates with the lower end of the suction passageway 32. The air pump unit 60 has a motor 61. The motor 61 is connected to the control board 20.

The present invention further discloses a control method of the foregoing nasal aspirator capable of effectively preventing backflow. After the control board 20 is powered on, the air pump unit 60 is turned on, and the motor 61 starts to run. The air pump unit 60 generates a suction to suck the air in the suction passageway 32, the chamber 31, the cavity 401, the through hole 502, the accommodating chamber 501, the first passageway 402 and the second passageway 403 in sequence. After the upper end of the upper suction nozzle 40 (i.e. the suction head 45) is placed in a user's nasal cavity, the secretions in the nasal cavity flow through the third passageway 405, the second passageway 403 and the first passageway 402 in sequence under the action of the suction and flow into the accommodating chamber 501 to be collected. When the motor 61 runs, the control board 20 controls the detection system 70 to detect in real time whether there is a liquid flowing into the chamber 31 of the lower suction nozzle 30 for performing backflow detection. If the liquid is detected, the control board 20 controls the motor 61 to stop running, or, the motor 61 continues to run for collecting the secretions.

## Claims

1. A nasal aspirator,: the nasal aspirator comprises a casing (10), a lower suction nozzle (30), an upper suction nozzle (40), a liquid storage cup (50) and an air pump unit (60); the lower suction nozzle (30) is disposed on an upper end of the casing (10), the lower suction nozzle (30) has a chamber (31) and a suction passageway (32) therein, the chamber (31) has an opening facing upward, an upper end of the suction passageway (32) communicates with the chamber (31); the upper suction nozzle (40) is mounted to an upper end of the lower suction nozzle (30), the upper suction nozzle (40) includes a main body shell (41), an inner tube (42) and an outer tube (43), a lower end of the main body shell (41) is hermetically connected to the upper end of the lower suction nozzle (30), the main body shell (41) has a cavity (401) with an opening facing downward, the cavity (401) communicates with the chamber (31), the inner tube (42) extends from a top wall of the cavity (401), the inner tube (42) has a first passageway (402), the outer tube (43) extends upward from an upper end face of the main body shell (41), the outer tube (43) has a second passageway (403), the second passageway (403) communicates with the first passageway (402); the liquid storage cup (50) is placed in the chamber (31) and the cavity (401), the liquid storage cup (50) includes a cup body (51) and a cup cover (52), the cup body (51) has an accommodating chamber (501) with an opening facing upward, the cup cover (52) is disposed on the cup body (51) to cover the opening of the accommodating chamber (501), the cup cover (52) is formed with a mounting hole (503), the inner tube (42) passes through the mounting hole (503) and extends into the accommodating chamber (501);
the air pump unit (60) is disposed in the casing (10), an air intake end of the air pump unit (60) communicates with a lower end of the suction passageway (32), the air pump unit (60) has a motor (61),
wherein the lower suction nozzle (30) has a first boss (33) extending from a bottom of the chamber (31), and the lower suction nozzle (30) further has a connecting tube (34) extending downward from a bottom of the lower suction nozzle (30), and the suction passageway (32) is formed in the first boss (33) and the connecting tube (34),
**characterized in that**
the cup cover (52) is formed with a through hole (502), the through hole (502) communicates with the accommodating chamber (501) and the cavity (401), an outer wall of the inner tube (42) is hermetically mated with an inner wall of the mounting hole (503);
the nasal aspirator comprises a control board (20), the control board (20) is disposed in the casing (10); a detection system (70) for detecting a liquid is provided in the chamber (31), the detection system (70) is connected to the control board (20), and the motor (61) is connected to the control board (20); and
the cup cover (52) is coupled to the cup body (51), a positioning post (506) extends upward from an upper end of the cup body (51), the cup cover (52) has a positioning hole (507), and the positioning post (506) is inserted through the positioning hole (507) and extends out of the cup cover (52).

2. The nasal aspirator as claimed in claim 1, wherein the lower end of the main body shell (41) is sleeved on the upper end of the lower suction nozzle (30), and a sealing ring (44) is sandwiched between an inner wall of the lower end of the main body shell (41) and an outer wall of the upper end of the lower suction nozzle (30).

3. The nasal aspirator as claimed in claim 1, wherein a waterproof and breathable membrane (35) is fixed on an upper end face of the first boss (33), and the waterproof and breathable membrane (35) covers an upper opening of the suction passageway (32).

4. The nasal aspirator as claimed in claim 1, wherein an outer bottom surface of the cup body (51) has a recess (504), a second boss (505) is formed on an inner bottom surface of the cup body (51), and the first boss (33) is located in the recess (504).

5. The nasal aspirator as claimed in claim 4, wherein the inner tube (42) is close to the second boss (505), and a lower opening of the first passageway (402) faces an upper surface of the second boss (505).

6. The nasal aspirator as claimed in claim 1, wherein a bottom of the chamber (31) is formed with a fixing hole (37), the fixing hole (37) extends downward and passes through a bottom of the lower suction nozzle (30), the detection system (70) is a probe, the probe is inserted and secured in the fixing hole (37), and the detection system (70) includes two detection systems connected to the control board (20).

7. The nasal aspirator as claimed in claim 1, wherein the outer tube (43) is provided with a detachable suction head (45), the suction head (45) has a third passageway (405) therein, the third passageway (405) communicates with the second passageway (403), and the upper suction nozzle (40) is provided with a detachable cover (80).

8. A control method of the nasal aspirator as claimed in claim 1, wherein after the control board (20) is powered on, the air pump unit (60) is turned on and the motor (61) starts to run, the air pump unit (60) generates a suction to suck air in the suction passageway (32), the chamber (31), the cavity (401), the through hole (502), the accommodating chamber (501), the first passageway (402) and the second passageway (403) in sequence, after an upper end of the upper suction nozzle (40) is placed in a user's nasal cavity, secretions in the nasal cavity flows through the second passageway (403) and the first passageway (402) in sequence under the action of the suction and flows into the accommodating chamber (501) to be collected, when the motor (61) runs, the control board (20) controls the detection system (70) to detect in real time whether there is the liquid flowing into the chamber (31) of the lower suction nozzle (30) for performing backflow detection, if the liquid is detected, the control board (20) controls the motor (61) to stop running, or, the motor (61) continues to run for collecting the secretions.

## Patentansprüche

1. - Ein Nasensauger: der Nasensauger umfasst ein Gehäuse (10), eine untere Saugdüse (30), eine obere Saugdüse (40), einen Flüssigkeitsspeicherbecher (50) und eine Luftpumpeneinheit (60); die untere Saugdüse (30) ist an einem oberen Ende des Gehäuses (10) angeordnet, die untere Saugdüse (30) hat eine Kammer (31) und einen Saugdurchgang (32) darin, die Kammer (31) hat eine nach oben gerichtete Öffnung, ein oberes Ende des Saugdurchgangs (32) steht mit der Kammer (31) in Verbindung; die obere Saugdüse (40) an einem oberen Ende der unteren Saugdüse (30) angebracht ist, die obere Saugdüse (40) ein Hauptkörpergehäuse (41), ein Innenrohr (42) und ein Außenrohr (43) aufweist, ein unteres Ende des Hauptkörpergehäuses (41) hermetisch mit dem oberen Ende der unteren Saugdüse (30) verbunden ist, das Hauptkörpergehäuse (41) einen Hohlraum (401) mit einer nach unten gerichteten Öffnung aufweist, der Hohlraum (401) steht mit der Kammer (31) in Verbindung, das innere Rohr (42) erstreckt sich von einer oberen Wand des Hohlraums (401), das innere Rohr (42) hat einen ersten Durchgang (402), das äußere Rohr (43) erstreckt sich von einer oberen Endfläche des Hauptkörpergehäuses (41) nach oben, das Außenrohr (43) hat einen zweiten Durchgang (403), der zweite Durchgang (403) steht mit dem ersten Durchgang (402) in Verbindung; der Flüssigkeitsspeicherbecher (50) in der Kammer (31) und dem Hohlraum (401) angeordnet ist, der Flüssigkeitsspeicherbecher (50) einen Becherkörper (51) und eine Becherabdeckung (52) aufweist, der Becherkörper (51) eine Aufnahmekammer (501) mit einer nach oben weisenden Öffnung aufweist, die Becherabdeckung (52) auf dem Becherkörper (51) angeordnet ist, um die Öffnung der Aufnahmekammer (501) abzudecken, die Becherabdeckung (52) mit einem Montageloch (503) ausgebildet ist, das Innenrohr (42) durch das Montageloch (503) hindurchgeht und sich in die Aufnahmekammer (501) erstreckt; die Luftpumpeneinheit (60) in dem Gehäuse (10) angeordnet ist, ein Lufteinlassende der Luftpumpeneinheit (60) mit einem unteren Ende des Saugdurchgangs (32) in Verbindung steht, die Luftpumpeneinheit (60) einen Motor (61) aufweist,
wobei die untere Saugdüse (30) einen ersten Vorsprung (33) aufweist, der sich von einem Boden der Kammer (31) erstreckt, und die untere Saugdüse (30) ferner ein Verbindungsrohr (34) aufweist, das sich von einem Boden der unteren Saugdüse (30) nach unten erstreckt, und der Saugdurchgang (32) in dem ersten Vorsprung (33) und dem Verbindungsrohr (34) ausgebildet ist,
**dadurch gekennzeichnet, dass**
die Becherabdeckung (52) mit einem Durchgangsloch (502) ausgebildet ist, das Durchgangsloch (502) mit der Aufnahmekammer (501) und dem Hohlraum (401) in Verbindung steht, eine Außenwand des Innenrohrs (42) hermetisch mit einer Innenwand des Montagelochs (503) verbunden ist;
der Nasensauger eine Steuerplatine (20) umfasst, wobei die Steuerplatine (20) in dem Gehäuse (10) angeordnet ist; ein Erfassungssystem (70) zum Erfassen einer Flüssigkeit in der Kammer (31) vorgesehen ist, das Erfassungssystem (70) mit der Steuerplatine (20) verbunden ist und der Motor (61) mit der Steuerplatine (20) verbunden ist; und
die Becherabdeckung (52) mit dem Becherkörper (51) gekoppelt ist, ein Positionierungspfosten (506) sich von einem oberen Ende des Becherkörpers (51) nach oben erstreckt, die Becherabdeckung (52) ein Positionierungsloch (507) aufweist und der Positionierungspfosten (506) durch das Positionierungsloch (507) eingeführt ist und sich aus der Becherabdeckung (52) heraus erstreckt.

2. - Nasensauger nach Anspruch 1, wobei das untere Ende des Hauptkörpers (41) auf das obere Ende des unteren Saugdüse (30) aufgeschoben ist und ein Dichtungsring (44) zwischen einer Innenwand des unteren Endes des Hauptkörpergehauses (41) und einer Außenwand des oberen Endes des unteren Saugdüse (30) angeordnet ist.

3. - Nasensauger nach Anspruch 1, wobei eine wasserdichte und atmungsaktive Membran (35) an einer oberen Endfläche des ersten Vorsprungs (33) befestigt ist und die wasserdichte und atmungsaktive Membran (35) eine obere Öffnung des Saugdurchgangs (32) abdeckt.

4. - Nasensauger nach Anspruch 1, wobei eine äußere Bodenfläche des Becherkörpers (51) eine Ausnehmung (504) aufweist, ein zweiter Vorsprung (505) an einer inneren Bodenfläche des Becherkörpers (51) ausgebildet ist und der erste Vorsprung (33) in der Ausnehmung (504) angeordnet ist.

5. - Nasensauger nach Anspruch 4, wobei das Innenrohr (42) nahe dem zweiten Vorsprung (505) liegt und eine untere Öffnung des ersten Durchgangs (402) einer oberen Fläche des zweiten Vorsprungs (505) gegenüberliegt.

6. - Nasensauger nach Anspruch 1, wobei ein Boden der Kammer (31) mit einem Befestigungsloch (37) ausgebildet ist, das Befestigungsloch (37) sich nach unten erstreckt und durch einen Boden der unteren Saugdüse (30) hindurchgeht, das Erfassungssystem (70) eine Sonde ist, die Sonde in das Befestigungsloch (37) eingeführt und befestigt ist und das Erfassungssystem (70) zwei mit der Steuerplatine (20) verbundene Erfassungssysteme umfasst.

7. - Nasensauger nach Anspruch 1, wobei das Außenrohr (43) mit einem abnehmbaren Saugkopf (45) versehen ist, der Saugkopf (45) einen dritten Durchgang (405) darin aufweist, der dritte Durchgang (405) mit dem zweiten Durchgang (403) in Verbindung steht und die obere Saugdüse (40) mit einer abnehmbaren Abdeckung (80) versehen ist.

8. - Steuerungsverfahren für den Nasensauger nach Anspruch 1, wobei nach dem Einschalten der Steuerplatine (20), dem Einschalten der Luftpumpeneinheit (60) und dem Anlaufen des Motors (61) die Luftpumpeneinheit (60) einen Sog erzeugt, um Luft in den Saugdurchgang (32) zu saugen, die Kammer (31), den Hohlraum (401), das Durchgangsloch (502), die Aufnahmekammer (501), den ersten Durchgang (402) und den zweiten Durchgang (403) nacheinander anzusaugen, nachdem ein oberes Ende der oberen Saugdüse (40) in die Nasenhöhle eines Benutzers eingeführt wurde, Sekrete in der Nasenhöhle unter der Wirkung des Sogs nacheinander durch den zweiten Durchgang (403) und den ersten Durchgang (402) fließen und in die Aufnahmekammer (501) fließen, um gesammelt zu werden, wenn der Motor (61) läuft, die Steuerplatine (20) das Detektionssystem (70) steuert, um in Echtzeit zu detektieren, ob die Flüssigkeit in die Kammer (31) der unteren Saugdüse (30) fließt, um eine Rückflussdetektion durchzuführen, wenn die Flüssigkeit detektiert wird, steuert die Steuerplatine (20) den Motor (61), um den Betrieb zu stoppen, oder der Motor (61) läuft weiter, um die Sekrete zu sammeln.

## Revendications

1. - Aspirateur nasal : l'aspirateur nasal comprend un boîtier (10), une buse d'aspiration inférieure (30), une buse d'aspiration supérieure (40), une coupelle de stockage de liquide (50) et une unité de pompe à air (60) ; la buse d'aspiration inférieure (30) est disposée sur une extrémité supérieure du boîtier (10), la buse d'aspiration inférieure (30) a une chambre (31) et un passage d'aspiration (32) à l'intérieur de celle-ci, la chambre (31) a une ouverture orientée vers le haut, une extrémité supérieure du passage d'aspiration (32) communique avec la chambre (31) ; la buse d'aspiration supérieure (40) est montée sur une extrémité supérieure de la buse d'aspiration inférieure (30), la buse d'aspiration supérieure (40) comprend une coque de corps principal (41), un tube intérieur (42) et un tube extérieur (43), une extrémité inférieure de la coque de corps principal (41) est hermétiquement reliée à l'extrémité supérieure de la buse d'aspiration inférieure (30), la coque de corps principal (41) a une cavité (401) avec une ouverture orientée vers le bas, la cavité (401) communique avec la chambre (31), le tube intérieur (42) s'étend à partir d'une paroi supérieure de la cavité (401), le tube intérieur (42) a un premier passage (402), le tube extérieur (43) s'étend vers le haut à partir d'une face d'extrémité supérieure de la coque de corps principal (41), le tube extérieur (43) a un deuxième passage (403), le deuxième passage (403) communique avec le premier passage (402) ; la coupelle de stockage de liquide (50) est placée dans la chambre (31) et la cavité (401), la coupelle de stockage de liquide (50) comprend un corps de coupelle (51) et un couvercle de coupelle (52), le corps de coupelle (51) a une chambre de réception (501) avec une ouverture orientée vers le haut, le couvercle de coupelle (52) est disposé sur le corps de coupelle (51) pour couvrir l'ouverture de la chambre de réception (501), le couvercle de coupelle (52) comporte un trou de montage (503), le tube intérieur (42) passe à travers le trou de montage (503) et s'étend dans la chambre de réception (501) ; l'unité de pompe à air (60) est disposée dans le boîtier (10), une extrémité d'admission d'air de l'unité de pompe à air (60) communique avec une extrémité inférieure du passage d'aspiration (32), l'unité de pompe à air (60) a un moteur (61),
la buse d'aspiration inférieure (30) ayant un premier bossage (33) s'étendant à partir d'un fond de la chambre (31), et la buse d'aspiration inférieure (30) a également un tube de liaison (34) s'étendant vers le bas à partir d'un fond de la buse d'aspiration inférieure (30), et le passage d'aspiration (32) est formé dans le premier bossage (33) et le tube de liaison (34),
**caractérisé par le fait que**
le couvercle de coupelle (52) comporte un trou traversant (502), le trou traversant (502) communique avec la chambre de réception (501) et la cavité (401), une paroi extérieure du tube intérieur (42) est hermétiquement couplée à une paroi intérieure du trou de montage (503) ;
l'aspirateur nasal comprend une carte de commande (20), la carte de commande (20) est disposée dans le boîtier (10) ; un système de détection (70) pour détecter un liquide est prévu dans la chambre (31), le système de détection (70) est connecté à la carte de commande (20), et le moteur (61) est connecté à la carte de commande (20) ; et
le couvercle de coupelle (52) est couplé au corps de coupelle (51), une tige de positionnement (506) s'étend vers le haut à partir d'une extrémité supérieure du corps de coupelle (51), le couvercle de coupelle (52) a un trou de positionnement (507), et la tige de positionnement (506) est introduite à travers le trou de positionnement (507) et s'étend hors du couvercle de coupelle (52).

2. - Aspirateur nasal selon la revendication 1, dans lequel l'extrémité inférieure de la coque de corps principal (41) est emmanchée sur l'extrémité supérieure de la buse d'aspiration inférieure (30), et une bague d'étanchéité (44) est prise en sandwich entre une paroi intérieure de l'extrémité inférieure de la coque de corps principal (41) et une paroi extérieure de l'extrémité supérieure de la buse d'aspiration inférieure (30).

3. - Aspirateur nasal selon la revendication 1, dans lequel une membrane imperméable à l'eau et respirante (35) est fixée sur une face d'extrémité supérieure du premier bossage (33), et la membrane imperméable à l'eau et respirante (35) couvre une ouverture supérieure du passage d'aspiration (32).

4. - Aspirateur nasal selon la revendication 1, dans lequel une surface inférieure extérieure du corps de coupelle (51) a un renfoncement (504), un second bossage (505) est formé sur une surface inférieure intérieure du corps de coupelle (51), et le premier bossage (33) est situé dans le renfoncement (504).

5. - Aspirateur nasal selon la revendication 4, dans lequel le tube intérieur (42) est proche du second bossage (505), et une ouverture inférieure du premier passage (402) fait face à une surface supérieure du second bossage (505).

6. - Aspirateur nasal selon la revendication 1, dans lequel un fond de la chambre (31) comporte un trou de fixation (37), le trou de fixation (37) s'étend vers le bas et passe à travers un fond de la buse d'aspiration inférieure (30), le système de détection (70) est une sonde, la sonde est introduite et fixée dans le trou de fixation (37), et le système de détection (70) comprend deux systèmes de détection connectés à la carte de commande (20).

7. - Aspirateur nasal selon la revendication 1, dans lequel le tube extérieur (43) comporte une tête d'aspiration (45) amovible, la tête d'aspiration (45) a un troisième passage (405) à l'intérieur de celle-ci, le troisième passage (405) communique avec le deuxième passage (403), et la buse d'aspiration supérieure (40) comporte un couvercle amovible (80).

8. - Procédé de commande de l'aspirateur nasal selon la revendication 1, dans lequel, après la mise sous tension de la carte de commande (20), l'unité de pompe à air (60) est mise sous tension et le moteur (61) commence à fonctionner, l'unité de pompe à air (60) génère une aspiration pour aspirer de l'air dans le passage d'aspiration (32), la chambre (31), la cavité (401), le trou traversant (502), la chambre de réception (501), le premier passage (402) et le deuxième passage (403), dans l'ordre, après qu'une extrémité supérieure de la buse d'aspiration supérieure (40) a été placée dans la cavité nasale d'un utilisateur, des sécrétions dans la cavité nasale s'écoulent à travers le deuxième passage (403) et le premier passage (402) dans l'ordre sous l'action de l'aspiration et s'écoulent dans la chambre de réception (501) pour être recueillies, lorsque le moteur (61) fonctionne, la carte de commande (20) commande le système de détection (70) pour détecter en temps réel si du liquide s'écoule ou non dans la chambre (31) de la buse d'aspiration inférieure (30) afin d'effectuer une détection de reflux ; si le liquide est détecté, la carte de commande (20) commande l'arrêt du moteur (61), ou le moteur (61) continue de fonctionner pour recueillir les sécrétions.
